# EUROPEAN PATENT APPLICATION

(11) **EP 4 454 703 A2**
(43) Date of publication of application: **30.10.2024**
(21) Application number: 24200515.5
(22) Date of filing: 15.10.2021
(51) Int. Cl.: A61P 3/10

(54) **PHARMACEUTICAL COMPOSITIONS WITH LOW AMOUNTS OF NITROSAMINE IMPURITIES AND METHODS FOR PRODUCING THE SAME**

(30) Priority: 16.10.2020 GB 202016450
(62) Divisional of application: 21801023.9
(71) Applicant: Zentiva K.S., 102 37 Praha 10 (CZ)
(72) Inventor: RIHA, Jaroslav, 107 00 Praha 10 (CZ); RIDVAN, Ludek, 102 00 Praha 10 (CZ); DOUSA, Michal, 339 01 Klatovy (CZ); KUBELKA, Tomas, 190 00 Praha 9 (CZ); GIBALA, Petr, Praha 9 (CZ); BARTACEK, Frantisek, 250 92 Sestajovice (CZ); STASIAK, Pawel, 80-180 Gdansk (PL); KALASEK, Stanislav, 592 42 Jimramov (CZ); KLUK, Anna, 10-691 Olsztyn (PL); ZVONÍEK, Vít, 170 00 Praha 7 Holesovice (CZ)
(74) Representative: Reddie & Grose LLP

(57) **Abstract**

The present invention relates to pharmaceutical compositions comprising metformin with low amounts of nitrosamine impurities and methods for producing the same. More specifically, the present invention relates to methods and compositions that reduce the amount of nitrosable compounds in the active pharmaceutical ingredient and/or reduce the conversion of nitrosable compounds into nitrosamines in the finished dosage form.

## Description

### Field of the Invention

The present invention relates to pharmaceutical compositions with low amounts of nitrosamine impurities and methods for producing the same. More specifically, the present invention relates to methods and compositions that reduce the amount of nitrosable compounds in the active pharmaceutical ingredient and/or reduce the conversion of nitrosable compounds into nitrosamines in the finished dosage form. This invention also relates to pharmaceutical compositions comprising metformin and combined pharmaceutical preparations comprising metformin and a sodium-glucose co-transporter-2 (SGLT2) inhibitor and/or a dipeptidyl peptidase-4 (DPP-4) inhibitor together with their use in the treatment of type !! diabetes.

### Background

Nitrosamines, such as N-nitrosodiethylamine (NDEA), N-nitrosodimethylamine (NDMA) and N-nitrosodiisopropylamine (NDIPA) are highly potent human carcinogens. Exposure to these compounds, even in small amounts, poses a significant risk to patients. Accordingly, acceptable daily limits of NDMA and NDEA have been set at 96 ng/day and 26.5 ng/day respectively. Recently, the US Food and Drug Administration (FDA) and the European Medicines Agency (EMA) reported the detection of nitrosamine impurities, such as NDMA, in higher than acceptable levels in various drug products including the sartan class of angiotensin !! receptor blockers, ranitidine, nizatidine and most recently, metformin, used to treat type !! diabetes. As a result of this discovery, batches of the implicated drugs were recalled and the Marketing Authorisation Holders (MAH) launched investigations to determine the risks of the nitrosamine impurities and evaluate the circumstances and conditions in which they may form.

It is well established that nitrosamine impurities result from nitrosable compounds, for example primary, secondary or tertiary amines or quaternary ammonium salts, reacting with nitrosating agents such as nitrous acid (typically formed *in situ* from nitrites, such as sodium nitrite, under acidic conditions). Specifically, NDMA is formed when dimethylamine (DMA) is nitrosated by a nitrosating agent. Nitrosable compounds and nitrosating agents may be introduced into the active pharmaceutical ingredient or drug product at any stage during the manufacturing process, for example in the reactants and solvents used or through excipients in the pharmaceutical formulation. They may also arise through degradation of the active pharmaceutical ingredient during drug product storage.

The most common approach to reducing nitrosamine impurities in the active pharmaceutical ingredient and/or finished dosage form has been to adapt synthesis routes to exclude nitrosable compounds and nitrosating agents that could react to form nitrosamines thus contaminating the final drug product. Although this represents an "ideal world" solution it requires the exclusion of solvents and starting materials containing primary, secondary and tertiary amines or quaternary ammonium salts that may result in more complex and expensive synthesis routes. Therefore, there remains a need for methods and compositions to reduce or prevent the formation of nitrosamine impurities in active pharmaceutical ingredients and final drug products with minimal change to the existing synthetic routes.

### Summary of Invention

In a first aspect, the invention provides a pharmaceutical composition comprising an active pharmaceutical ingredient or salt thereof, wherein the composition comprises less than 100 ppm of peroxide impurities and more than 50 ppm of an amine impurity.

In a second aspect, the invention provides a pharmaceutical composition comprising an active pharmaceutical ingredient or salt thereof and a pH modifying agent, wherein the composition comprises more than 50 ppm of peroxide impurities and less than 50 ppm of an amine impurity.

In a third aspect, the invention provides a method for reducing the amount of an amine impurity in an active pharmaceutical ingredient or salt thereof, the method comprising:
a) providing a solution of the active pharmaceutical ingredient or salt thereof in a solvent;
b) crystallising the active pharmaceutical ingredient from the solvent; and
c) removing the solvent to obtain the pure active pharmaceutical ingredient or salt thereof.

In a fourth aspect, the invention provides a method for reducing the amount of an amine impurity in an active pharmaceutical ingredient or salt thereof, the method comprising: heating the active pharmaceutical ingredient or salt thereof to a temperature between 50 and 120 °C.

In another aspect, the invention provides a pharmaceutical composition comprising metformin or a salt thereof and an anti-oxidant, free radical scavenger or reducing agent, wherein the composition comprises less than 100 ppm of peroxide impurities and more than 50 ppm of dimethylamine, for use in the treatment of type !! diabetes.

In a further aspect, the present invention provides a combined pharmaceutical preparation comprising metformin or a salt thereof, an anti-oxidant, free radical scavenger or reducing agent and a sodium-glucose co-transporter-2 (SGLT2) inhibitor and/or a dipeptidyl peptidase-4 (DPP-4) inhibitor, wherein the composition comprises less than 100 ppm of peroxide impurities and more than 50 ppm of dimethylamine.

In a further aspect, the present invention provides a combined pharmaceutical preparation comprising metformin or a salt thereof, an anti-oxidant, free radical scavenger or reducing agent and a sodium-glucose co-transporter-2 (SGLT2) inhibitor and/or a dipeptidyl peptidase-4 (DPP-4) inhibitor, wherein the composition comprises less than 100 ppm of peroxide impurities and more than 50 ppm of dimethylamine, for use in the treatment of type !! diabetes.

Other features, embodiments and advantages will be apparent from the description and the claims.

### Detailed Description

As an alternative to attempting to reduce the presence of nitrosamine or nitrosable compound impurities during the synthesis of the active pharmaceutical ingredient, the present invention provides methods and compositions that reduce the amount of nitrosable compounds (e.g. amines) in the active pharmaceutical ingredient and/or reduce the conversion of nitrosable compounds into nitrosamines.

According to the present invention, in a first aspect there is provided a pharmaceutical composition comprising an active pharmaceutical ingredient or salt thereof (e.g. metformin hydrochloride), wherein the composition comprises less than 100 ppm (e.g. less than 80 ppm, less than 60 ppm, less than 40 ppm) of peroxide impurities (e.g. hydrogen peroxide) and more than 50 ppm (e.g. more than 70 ppm, more than 90 ppm, more than 110 ppm, or more than 130 ppm) of an amine impurity (e.g. a primary, secondary, or tertiary amine or quaternary ammonium salt). The amine impurity may be dimethylamine. Alternatively, the amine impurity may be from 50 to 500 ppm, from 60 to 400 ppm, from 70 to 300 ppm, or from 80 to 200 ppm.

As used herein the term "active pharmaceutical ingredient" refers to the substance (or combination of substances) that is the biologically active component of a drug product. As used herein the term "or salt thereof" refers to any crystalline or amorphous salt of the active pharmaceutical agent, prepared from bases or acids including inorganic or organic bases and inorganic and organic acids. As used herein the term "pharmaceutically acceptable salt" refers to any non-toxic salt, prepared from bases or acids including inorganic or organic bases and inorganic and organic acids, suitable for use in human drug products. The active pharmaceutical ingredient or salt thereof used in any aspect of the present invention may be any biologically active component of a drug product. Preferably the salt is a pharmaceutically acceptable salt (e.g. any pharmaceutically acceptable salt known in the art, e.g. hydrochloride or sulphate). The active pharmaceutical ingredient of salt thereof may be an angiotensin !! receptor blocker of the sartan class (e.g. valsartan), ranitidine, nizatidine or metformin (e.g. metformin hydrochloride). Preferably the active pharmaceutical ingredient or salt thereof is metformin (e.g., metformin hydrochloride). The active pharmaceutical ingredient or salt thereof may be prepared according to any process described in the literature.

As used herein the term "amine impurity" refers to any primary, secondary or tertiary amine or quaternary ammonium salt capable of being nitrosated by a nitrosating agent to form a nitrosamine. It will be appreciated that amine impurities may have been introduced into the active pharmaceutical ingredient and/or pharmaceutical composition at any stage during the manufacturing process, for example in the reactants and solvents used (i.e., dimethylamine, introduced during in the synthesis of metformin hydrochloride) or formed through the degradation of the active pharmaceutical ingredient or salt thereof. It will be further appreciated that one or more different amine impurities (e.g. primary, secondary, or tertiary amines or quaternary ammonium salts) may be present in a pharmaceutical composition according to the present invention. Thus the pharmaceutical composition may comprise one or more amine impurities or a combination thereof. Such amine impurities may include, but are not limited to, dimethylamine (DMA), diethylamine (DEA), triethylamine (TEA), diisopropylethylamine (DIPEA), dibutylamine (DBA), tributylamine (TBA) and N-methyl-4-aminobutyric acid (MBA).

As mentioned above, the formation of nitrosamine impurities may occur when a nitrosable compound (e.g. an amine impurity or ammonia) reacts with a nitrosating agent. One such way nitrosating agents may be introduced into a pharmaceutical composition is by the oxidation of amine and/or ammonia impurities by oxidizing agents, for example atmospheric oxygen or peroxide impurities present in the composition. As used herein the term "peroxide impurities" refers to any residual peroxide, for example hydrogen peroxide, present in a substance (e.g. an excipient) or in a pharmaceutical composition. Peroxide impurities may be introduced into a pharmaceutical composition through the presence of excipients, for example povidone, crospovidone or polysorbate. Peroxide impurities may react with amine and/or ammonia impurities in a pharmaceutical composition to form nitrosating agents. Additionally, peroxide impurities present in excipients may also react with the active pharmaceutical ingredient or salt thereof to form nitrosating agents. In drug products the presence of excipients such as binders, disintegrants or solubilsers (including those containing peroxide impurities) is necessary to, for example, facilitate disintegration of tablets, bind together the dry ingredients or help solubilize the active pharmaceutical ingredient.

The pharmaceutical composition according to the first aspect of the invention may comprise a low peroxide grade excipient. As used herein the term "low peroxide grade excipient" refers to an excipient with a low amount of peroxide impurities (e.g. less than 100 ppm, less than 80 ppm, less than 60 ppm, less than 40 ppm, less than 30 ppm). Low peroxide grade excipients may also prevent the formation of peroxide impurities themselves, for example through integrated anti-oxidants such as sulphites, and/or prevent oxidation, for example by atmospheric oxygen, of substances in the drug product. Low peroxide grade excipients may include, but are not limited to, excipients such as binders, disintegrants or solubilsers, for example polyvinylpyrrolidone (povidone), cross-linked polyvinylpyrrolidone (crospovidone) or polysorbate. Preferably the low peroxide grade excipient is povidone LP30 or povidone K25. The low peroxide grade excipients (e.g. povidone LP30 or povidone K25) may be commercially available or prepared according to any method known in the art to remove/reduce peroxide impurities from commercially available excipients.

The inventors have surprisingly found that in pharmaceutical compositions comprising nitrosable compounds (e.g. amine impurities or ammonia) and low peroxide grade excipients, the amount of the corresponding nitrosamine impurity formed in the final drug product is significantly reduced to an acceptable level. Such a finding is hugely advantageous because it negates the need to adapt synthesis routes to exclude nitrosable compounds and for additional purification steps to reduce the amount of amine impurities in the active pharmaceutical ingredient or salt thereof.

As used herein the term "acceptable level" of nitrosamine impurities refers to an amount of nitrosamine impurity that is not considered to pose significant risk to the patient based on the daily limits set by the EMA or FDA, for example, 96 ng/day for NDMA and 26.5 ng/day for NDEA (EMA/217823/2019 14 Feb 2019 angiotensin-II-receptor antagonists (sartans) containing a tetrazole). Further examples of nitrosamine impurities include, but are not limited to N-nitrosodimethylamine (NDMA), N-nitrosodiethylamine (NDEA), N-nitrosodiisopropylamine (DIPNA), N-nitrosoethylisopropylamine (EIPNA), N-nitrosodibutylamine (NDBA) and N-nitroso-N-methyl-4-aminobutyric acid (NMBA)). It will be appreciated that the acceptable level will vary depending on the nitrosamine impurity and where more than one nitrosamine impurity may be present the amount is calculated for each nitrosamine impurity independently. It will be appreciated that the amount of impurities (e.g. amine, nitrosamine, ammonia, peroxide) may be detected according to any technique known in the art, for example GC/MS. The pharmaceutical compositions of the invention may comprise less than 0.1 ppm of nitrosamine impurities, preferably less than 0.08 ppm, less than 0.06 ppm, less than 0.05 ppm, less than 0.04 ppm, less than 0.03 ppm, less than 0.02 ppm or less than 0.01 ppm.

The pharmaceutical composition according to the first aspect of the invention may comprise at least one of an anti-oxidant, free radical scavenger or reducing agent (e.g. sulphites such as sodium sulphite). The reducing agent, antioxidant or radical scavenger may be selected from a group consisting of sodium sulphite, sodium bisulphite, sodium metabisulphite, sodium thiosulphate, sodium formaldehyde sulfoxylate or a combination thereof. Preferably the reducing agent, antioxidant or radical scavenger is sodium sulphite. Where the pharmaceutical composition according to the first aspect of the invention comprises a low peroxide grade excipient, the anti-oxidant, free radical scavenger or reducing agent active may be integrated in the low peroxide grade excipient, for example sulphites integrated into povidone. Alternatively, the anti-oxidant, free radical scavenger or reducing agent may be separate and independent from the low peroxide grade excipient. The anti-oxidant, free radical scavenger or reducing agent may be present in an amount from 0.001 to 1 wt%, 0.05 to 0.5 wt%, 0.07 to 0.3 wt% or 0.09 to 0.1 wt% of the pharmaceutical composition.

The inventors have surprisingly found that in a pharmaceutical composition comprising nitrosable compounds (e.g. amine impurities or ammonia) and at least one of an anti-oxidant, reducing agent or radical scavenger, the amount of the corresponding nitrosamine impurity formed in the final drug product is significantly reduced to an acceptable level over the shelf-life of the product. Such a finding is hugely advantageous because it negates the need to adapt synthesis routes to exclude nitrosable compounds and for additional purification steps to reduce the amount of amine impurities in the active pharmaceutical ingredient or salt thereof.

In a second aspect of the present invention there is provided a pharmaceutical composition comprising an active pharmaceutical ingredient or salt thereof (e.g. metformin hydrochloride); and a pH modifying agent (e.g. sodium carbonate, sodium bicarbonate, sodium hydrogen carbonate), wherein the composition comprises more than 50 ppm (e.g. more than 60 ppm, more than 80 ppm, more than 100 ppm, or more than 120 ppm) of peroxide impurities (e.g. hydrogen peroxide) and less than 50 ppm (e.g. less than 40 ppm, less than 30 ppm, less than 20 ppm, or less than 10 ppm) of an amine impurity (e.g. a primary, secondary, or tertiary amine or quaternary ammonium salt).

As used herein the term "pH modifying agent" is an agent that is capable of modifying the pH of the composition to achieve a desired pH. Thus the pH modifying agent is capable of achieving a pH range ranging from mildly acidic to mildly alkaline, preferably the pH is from 2 to 8, e.g. pH from 3 to 7, pH from 4 to 6. The pH modifying agent may be selected from a group consisting of an acid, base, or buffer. Preferably the pH modifying agent is a base, for example a carbonate or hydrogen carbonate salt of an alkali or alkaline earth metal. Most preferably the pH modifying agent is sodium carbonate, sodium bicarbonate or sodium hydrogen carbonate. The pH modifying agent may be present in an amount from 0.1 to 5 wt%, 0.2 to 4 wt%, 0.3 to 3 wt%, 0.4 to 2 wt% or 0.5 to 1 wt% of the pharmaceutical composition.

It will be appreciated that peroxide impurities may be introduced into a pharmaceutical composition by excipients, for example povidone, crospovidone or polysorbate. The pharmaceutical composition according to the second aspect of the invention may comprise an excipient, for example a binder, disintegrant or solubilser. The binder, disintegrant or solubilser may be povidone, crospovidone or polysorbate. The excipient may contain peroxide impurities (e.g. hydrogen peroxide). The amount of peroxide impurities in the excipient may be more than 50 ppm, more than 60 ppm, more than 80 ppm, more than 100 ppm, or more than 120 ppm. Alternatively, the peroxide impurity may be from 50 to 250 ppm, from 60 to 240 ppm, from 70 to 220 ppm, from 80 to 200 ppm, or from 90 to 180 ppm.

The inventors have surprisingly found that in a pharmaceutical composition comprising amine and/or ammonia impurities and peroxide impurities (e.g. from excipients containing peroxide impurities such as povidone, crospovidone or polysorbate), the presence of pH modifying agents reduces the amount of an amine impurity and/or ammonia impurity, thus the amount of the corresponding nitrosamine impurity formed in the final drug product is significantly reduced to an acceptable level.

Thus the present invention allows for the presence of excipients containing peroxide impurities, such as binders, disintegrants or solubilsers in a pharmaceutical composition yet reduces the amount of nitrosamine impurities formed in the final drug product by way of reducing the amount of amine impurities (e.g. nitrosable compounds) in the composition. Accordingly, an acceptable level of nitrosamine impurities is maintained over the shelf-life of the product. Such a finding is hugely advantageous because it negates the need to adapt synthesis routes to exclude nitrosable compounds and for additional purification steps to reduce the amount of amine impurities in the active pharmaceutical ingredient or salt thereof.

The present invention further provides a method for reducing the amount of an amine impurity in a pharmaceutical composition, the method comprising:
a) mixing the active pharmaceutical ingredient or salt thereof with a pH modifying agent;
b) formulating the mixture of a) into a finished dosage form.

The active pharmaceutical ingredient or salt thereof and pH modifying agent may be the same as those referred to in relation to the second aspect of the invention. Further, the method may be used in the manufacture of the pharmaceutical compositions according to the second aspect of the invention.

Surprisingly it has been found that by using a pH modifying agent during the formulating process, excipients and active pharmaceutical ingredients with an amine impurity of greater than 50 ppm may be used as starting materials in the formulation process.

A third aspect of the present invention provides a method for reducing the amount of an amine impurity (e.g. a primary, secondary, or tertiary amine or quaternary ammonium salt) in an active pharmaceutical ingredient or salt thereof (e.g. metformin hydrochloride), the method comprising:
a) providing a solution of the active pharmaceutical ingredient or salt thereof (e.g. metformin hydrochloride) in a solvent (e.g. water or a mixture of water and an alcoholic solvent e.g. water and methanol);
b) crystallising the active pharmaceutical ingredient or salt thereof (e.g. metformin hydrochloride) from the solvent (e.g. water or a mixture of water and an alcoholic solvent e.g. water and methanol); and
c) removing the solvent (e.g. by filtration) to obtain the pure active pharmaceutical ingredient or salt thereof (e.g. metformin hydrochloride).

As mentioned previously, nitrosamine impurities result from nitrosable compounds (e.g. amine impurities or ammonia) reacting with nitrosating agents such as nitrous acid (typically formed *in situ* from nitrites under acidic conditions). Thus the present invention provides a method for reducing the amount of a nitrosable compound and/or amine impurity (e.g. primary, secondary, or tertiary amines or quaternary ammonium salts) in the active pharmaceutical ingredient or salt thereof, thereby reducing the amount of the corresponding nitrosamine impurity in the final drug product.

As used herein the term "pure active pharmaceutical ingredient or salt thereof" refers to an active pharmaceutical ingredient of salt thereof comprising less 50 ppm of an amine impurity, less 40 ppm of an amine impurity, less than 30 ppm, less than 20 ppm or less than 10 ppm.

Preferably the solution of active pharmaceutical ingredient or salt thereof may be obtained by dissolving the active pharmaceutical ingredient or salt thereof in a solvent (e.g. water, an alcoholic solvent, or a mixture thereof). Optionally, the active pharmaceutical ingredient or salt thereof may be dissolved in a solvent at room temperature (e.g. from 16 to 25 °C) or by heating the mixture from, for example ambient to reflux temperature (e.g. from 25 to 120 °C, from 30 to 100 °C, or from 60 to 80 °C). The solvent may be selected from a group consisting of water, an alcoholic solvent, or a mixture thereof. Preferably the solvent is water or a mixture of water and an alcoholic solvent. The alcoholic solvent may be selected from primary, secondary or tertiary alcohols (e.g. methanol, ethanol, 2-propanol, or 2-butanol). Preferably the alcoholic solvent is a primary alcohol (e.g. methanol, ethanol, or propanol). Most preferably the alcoholic solvent is methanol. Where the solvent may be a combination of water and an alcoholic solvent, the ratio of the alcoholic solvent to water may be 1:1, 2:1, 3:1, 4:1 or 5:1. Preferably the ratio of the alcoholic solvent to water is 4:1.

Crystallisation of the active pharmaceutical ingredient from the solvent may be obtained by any method known in the art. Preferably crystallisation occurs by cooling the solution of the active pharmaceutical ingredient to a temperature from 0 to 45 °C (e.g. from 0 to 30 °C, from 5 to 25 °C, or from 10 to 20 °C) to precipitate the pure active pharmaceutical ingredient (i.e. the active pharmaceutical ingredient with reduced amounts of amine impurity). Optionally the solution of the active pharmaceutical ingredient may be seeded to promote crystal growth before cooling to a temperature from 0 to 45 °C (e.g. from 0 to 30 °C, from 5 to 25 °C, or from 10 to 20 °C) to precipitate the pure active pharmaceutical ingredient. The solvent may be removed from the solution to obtain the pure active pharmaceutical ingredient or salt thereof by any method known in the art, for example by evaporation of the solvent or by filtration. Preferably the solvent is removed by filtration. The crystals of pure active pharmaceutical ingredient may be dried under any suitable drying conditions such as aerial drying, vacuum drying, rotary vacuum drying or drying under inert gases (e.g. nitrogen). Optionally the drying may occur at a temperature of around 40 °C to 80 °C (e.g. 40 °C, 50 °C, 60 °C, 70 °C, or 80 °C) under reduced pressure (e.g. from 50 to 150 mbar) for at least one hour (e.g. from 1 to 24 hours, from 2 to 12 hours, from 3 to 6 hours).

It will be appreciated that the method according to the third aspect of the present invention may be used in the preparation of the active ingredients used in any of aspects one or two of the invention.

In a fourth aspect of the present invention there is provided a method for reducing the amount of an amine impurity (e.g. a primary, secondary, or tertiary amine or quaternary ammonium salt) in an active pharmaceutical ingredient or salt thereof (e.g. metformin hydrochloride), the method comprising: heating the active pharmaceutical ingredient or salt thereof (e.g. metformin hydrochloride) to a temperature between 50 and 120 °C (e.g. between 60 and 110 °C, between 70 and 100 °C, or between 80 and 90 °C).

The inventors have surprisingly found that the amount of amine impurities and/or ammonia impurities in the active pharmaceutical ingredient or salt thereof can be reduced by heating the active pharmaceutical ingredient or salt thereof with no subsequent degradation of the active pharmaceutical ingredient or salt thereof.

The active pharmaceutical ingredient or salt thereof may be heated by any method known in the art. Preferably the active pharmaceutical ingredient or salt thereof is heated in a drying oven. The active pharmaceutical ingredient or salt thereof may be heated under reduced pressure (e.g. from 50 to 150 mbar). The active pharmaceutical ingredient or salt thereof may be heated for at least one hour (e.g. from 1 to 24 hours, from 2 to 18 hours, from 3 to 12 hours or from 4 to 6 hours). The amine impurities and/or ammonia impurities are removed by sublimation (solid to vapour phase transition) at these elevated temperatures. Thus the method of the fourth aspect of the present invention reduces the amount of nitrosable compounds in the active pharmaceutical ingredient or salt thereof.

It will be appreciated that the method according to the fourth aspect of the present invention may be used in combination with the method described in the third aspect of the invention or in preparation of the active ingredients used in any of aspects one or two of the invention.

The pharmaceutical compositions of the invention may be for use in the treatment of type II diabetes. Optionally, the pharmaceutical compositions are for oral administration. Preferably, the pharmaceutical compositions are in tablet form. The tablet may be a coated tablet, for example a film-coated tablet. The pharmaceutical compositions of the invention may be for use in (or in the manufacture of medicaments for) the treatment of type II diabetes. In a further aspect of the invention, there is provided a method of treatment of type !! diabetes comprising: administering to a patient in need thereof a pharmaceutical composition according to the present invention, as set out above.

The pharmaceutical compositions of the invention may be combined with an anti-diabetic compound in a combined pharmaceutical preparation. The anti-diabetic compound may be a sodium-glucose co-transporter-2 (SGLT2) inhibitor and/or a dipeptidyl peptidase-4 (DPP-4) inhibitor. The sodium-glucose co-transporter-2 (SGLT2) inhibitor may be a flozin e.g., Empagliflozin, Dapagliflozin, Canagliflozin. The dipeptidyl peptidase-4 (DPP-4) inhibitor may be a gliptin e.g., Vildagliptin, Sitagliptin, Saxagliptin, Alogliptin, Linagliptin. Preferably the dipeptidyl peptidase-4 (DPP-4) inhibitor is Sitagliptin or Linagliptin. It will be appreciated the pharmaceutical compositions of the invention may be combined with more than one anti-diabetic compound in a combined pharmaceutical preparation.

According to the present invention, there is also provided a combined pharmaceutical preparation comprising metformin or a salt thereof, an anti-oxidant, free radical scavenger or reducing agent, and a sodium-glucose co-transporter-2 (SGLT2) inhibitor and/or a dipeptidyl peptidase-4 (DPP-4) inhibitor, wherein the composition comprises less than 100 ppm of peroxide impurities and more than 50 ppm of dimethylamine.

Combined pharmaceutical preparations comprising metformin and a flozin or gliptin are commercially available, for example under the brand names Synjardy (RTM) (Empagliflozin and metformin), Janumet (RTM) (Sitagliptin and metformin), Jentadueto (Linagliptin and metformin) and Vokanamet (RTM) (Canagliflozin and metformin). However, such combined preparations may comprise NDMA in higher than acceptable levels (i.e., above the daily limit). Advantageously, the combined pharmaceutical preparation of the present invention provides a combined metformin preparation (e.g., Sitagliptin and metformin, e.g., Linagliptin and metformin) with an acceptable amount of NDMA, which is below the acceptable daily limit.

The combined pharmaceutical preparations may be for use in the treatment of type !! diabetes. Optionally, the combined pharmaceutical preparations are for oral administration. Preferably, the combined pharmaceutical preparations are in tablet form. The tablet may be a coated tablet, for example a film-coated tablet. The combined pharmaceutical preparations of the invention may be for use in (or in the manufacture of medicaments for) the treatment of type !! diabetes. In a further aspect of the invention, there is provided a method of treatment of type !! diabetes comprising: administering to a patient in need thereof a combined pharmaceutical preparation according to the present invention, as set out above.

The present invention is now described in more detail by, but is not limited to, the following Examples.

### Examples

### Recrystallisation of Metformin Hydrochloride (HCl)

In Example 1a and 1b described below, metformin HCl containing 228 ppm of dimethylamine (DMA) impurity was used as the starting material.

### Example 1a

Metformin HCl (15 g) was dissolved in 4:1 methanol: water mixture (50 ml) at reflux. After all the metformin HCl was dissolved, the reaction mixture was cooled slowly to 10 °C to precipitate solid metformin HCl. The metformin HCl precipitate was filtered off and dried at 40 °C and 150 mbar for 16 hours. Yield: 11.0 g (73 %) and DMA content 16 ppm.

### Example 1b

Metformin HCl (8.3 kg) was dissolved in water (14 L) at 60 °C. After all the metformin HCl was dissolved, the reaction mixture was cooled to 45 °C and seeded with pure metformin HCl (45 g) to initiate crystallisation. The reaction mixture was cooled slowly to 10 °C to precipitate solid metformin HCl. The metformin HCl precipitate was filtered off, washed with water (1.3 L) and dried at 40 °C and 150 mbar for 16 hours. Yield: 5.1 g (61 %) and DMA content < 5 ppm.

### Drying of Metformin Hydrochloride (HCl)

In Example 2 described below, metformin HCl containing 228 ppm of DMA impurity was used.

### Example 2

Samples of metformin HCl (5 g) were put in a drying oven at temperatures of 50, 75 or 90 °C. The DMA content of the metformin HCl samples was measured after 6 and 24 hours. The results are summarised in Table 1.

**Table 1: DMA content of batches of metformin HCl after 6 and 24 hours after drying at 50, 75 or 90 °C**

| | **Temperature (°C)** | | |
|---|---|---|---|
| **DMA content (ppm)** | **50** | **75** | **90** |
| **After 6 hours** | 150 ppm | 68 ppm | 29 ppm |
| **After 24 hours** | 82 ppm | 28 ppm | 21 ppm |

### Formulation Experiments with Metformin HCl

The following examples describe formulation experiments to reduce the amount of nitrosable compounds and/or reduce the conversion of nitrosable compounds into nitrosamines in compositions comprising metformin HCl.

### General Preparation Procedure

All formulation experiments were performed according to the general preparation procedure described below, unless otherwise specified:
Metformin HCl (400.0 g) was loaded into a high shear granulator and chopped for 20 minutes. Hypromellose 15 MPa-s (14.08 g) was added and chopped with the metformin HCl for an additional 10 minutes. A solution of povidone K25 (21.20 g) in water (30.00 g) was pumped into the high shear granulator over a 9 minute period and kneaded for an additional 4 minutes. The obtained granules were dried using fluid-bed drying, to achieve a water content of below 1.4%. The granules were then sieved, lubricated with magnesium stearate (2.32 g) and subjected to compression (12.0 mm diameter, 20R, tablet thickness, 5.10 mm, 547 mg/tablet). The tablets were then film-coated with a suspension of Hypromellose 5 MPa-s (5.20 g), macrogol 6000 (1.04 g) and titanium dioxide (4.16 g) in water (57 g) using a pan coater. The amount of coating in each individual tablet was 13 mg.

### Formulation of Metformin HCl with different amounts of DMA impurity

The formulations described below (Examples 3 to 5) were prepared using different batches of metformin HCl. Each batch contained a different amount of DMA impurity. The batches of metformin hydrochloride were either used directly from suppliers (i.e. with no further purification) or prepared according to the methods described in Examples 1a or 1b above.

### Example 3

Metformin HCl containing 228 ppm of DMA impurity was used as the starting material. The batch was then prepared substantially as described in the general preparation procedure above.

### Example 4

Metformin HCl containing < 5 ppm of DMA impurity, prepared according to the method of Example 1b above, was used as the starting material. The batch was then prepared substantially as described in the general preparation procedure above.

### Example 5

Metformin HCl containing 78 ppm of DMA impurity was used as the starting material. The batch was then prepared substantially as described in the general preparation procedure above.

The NDMA content of the batches in Examples 3 to 5 under various conditions, including under stress, was then measured. The NDMA content was measured immediately after formulation (T0), after 57 days storage in a glass bottle in laboratory conditions and after 7 days storage at 60 °C and 75 % relative humidity. The results are summarised in Table 2.

**Table 2: The NDMA content of batches of metformin HCl containing different amounts of DMA impurity, measured immediately after formulation, after 57 days storage in a glass bottle in laboratory conditions and after 7 days storage at 60 °C and 75 % relative humidity**

| | **NDMA Content (ppm)** | | |
|---|---|---|---|
| **Batch** | T0 | lab. cond, glass bottle, 57 days | 60 °C / 75 % RH, AI/AI, 7 days |
| **Example 3** | 0.014 | 0.069 | 0.062 |
| **Example 4** | < 0.008 | 0.009 | 0.018 |
| **Example 5** | < 0.008 | 0.018 | 0.031 |

For metformin HCl, it is desirable to keep the amount of DMA impurity less than 50 ppm and the NDMA impurity less than 0.05 ppm.

### Formulation of Metformin HCl with low peroxide grade excipients

The inventors investigated the effect that the amount of peroxide impurities has on NDMA formation in a drug product comprising metformin HCl.

The formulations described below (Examples 6 to 7) were prepared using metformin HCl and a low peroxide grade povidone, i.e. povidone with a low amount of peroxide impurities, for example, povidone containing less than 100 ppm of peroxide.

### Example 6

The batch was prepared substantially as described in the general preparation procedure above. Povidone K25 used for the preparation of this batch contained 23 ppm peroxide impurities.

### Example 7

The batch was prepared substantially as described in the general preparation procedure above. Povidone K25 used for the preparation of this batch contained 86 ppm peroxide impurities.

The NDMA content of the batches in Examples 6 and 7 was then measured. The results are summarised in Table 3.

**Table 3: The NDMA content of batches containing metformin HCl with different amounts of peroxide impurity**

| **Batch** | **NDMA (ppm)** |
|---|---|
| **Example 6** | 0.008 |
| **Example 7** | 0.015 |

### Formulation of Metformin HCl with excipients with integrated anti-oxidants

The inventors investigated the effect of excipients with integrated anti-oxidants on the NDMA content of the finished dosage form in compositions comprising metformin HCl. Specifically, the effect of povidone LP30, a povidone with an integrated sulphite-based anti-oxidant, was investigated. The sulphite content in povidone LP30 (BASF) is typically between 200 to 300 ppm.

The following formulation experiments (Examples 8 to 11) were performed according to the general preparation procedure described below:
Metformin HCl (4000 g) was loaded into a high shear granulator and chopped for 20 minutes. Hypromellose 15 MPa-s (140.8 g) was added and chopped with the metformin HCl for an additional 10 minutes. A solution of povidone K25 or povidone LP30 (212.0 g) in water (300.0 g) was pumped into the high shear granulator over a 9 minute period and kneaded for an additional 4 minutes. The obtained granules were dried using fluid-bed drying, to achieve a water content of below 1.4%. The granules were then sieved, lubricated with magnesium stearate (23.2 g) and subjected to compression (oblong 18.4 mm x 9.4 mm, tablet thickness, 7.10 mm, 1094 mg/tablet). The tablets were then film-coated with a suspension of Hypromellose 5 MPa-s (48.00 g), macrogol 6000 (10.00 g) and titanium dioxide (38.00 g) in water (560 g) using a pan coater. The amount of coating in each individual tablet was 24 mg.

### Example 8

The batch was prepared substantially as described in the general preparation procedure above, using povidone LP30. Metformin HCl containing 228 ppm of DMA impurity was used as the starting material.

### Example 9

The batch was prepared substantially as described in the general preparation procedure above, using povidone K25. Metformin HCl containing 228 ppm of DMA impurity was used as the starting material.

### Example 10

The batch was prepared substantially as described in the general preparation procedure above, using povidone LP30. Metformin HCl containing 19 ppm of DMA impurity was used as the starting material.

### Example 11

The batch was prepared substantially as described in the general preparation procedure above, using povidone K25. Metformin HCl containing 19 ppm of DMA impurity was used as the starting material.

The NDMA content of Examples 8 to 11 under various conditions was then measured. The NDMA content of the batches was measured immediately after formulation (T0), after 7 days storage at 60 °C and 75 % relative humidity, after 1 month storage at 40 °C and 75 % relative humidity and after 1 month storage at 25 °C and 60 % relative humidity. The results are summarised in Table 4.

**Table 4: The NDMA content of batches containing metformin HCl with different amounts of DMA impurity and formulated with either povidone LP30 or povidone K25, measured immediately after formulation (T0), after 7 days storage at 60 °C and 75 % relative humidity, after 1 month storage at 40 °C and 75 % relative humidity and after 1 month storage at 25 °C and 60 % relative humidity**

| | **NDMA Content (ppm)** | | | |
|---|---|---|---|---|
| **Batch** | T0 | 60 °C / 75 % RH, AI/AI, 7 days | 40 °C / 75 % RH, PVC/AI, 1 month | 25 °C / 60 % RH, PVC/AI, 1 month |
| **Example 8** | < 0.010 | 0.011 | 0.011 | 0.012 |
| **Example 9** | 0.013 | 0.069 | 0.043 | 0.042 |
| **Example 10** | < 0.010 | < 0.010 | < 0.010 | < 0.010 |
| **Example 11** | < 0.010 | 0.031 | 0.017 | 0.017 |

### Two and Three Month Stability Results

The NDMA content of Examples 8 to 11 was measured after 2 and 3 months storage at 40 °C and 75 % relative humidity and after 2 and 3 months storage at 25 °C and 60 % relative humidity. The results are summarised in Table 5.

**Table 5: The NDMA content of batches containing metformin HCl with different amounts of DMA impurity and formulated with either povidone LP30 or povidone K25 measured after 2 and 3 months storage at 40 °C and 75 % relative humidity and after 2 and 3 months storage at 25 °C and 60 % relative humidity**

| | **NDMA Content (ppm)** | | | |
|---|---|---|---|---|
| **Batch** | 40 °C / 75 % RH, PVC/AI, 2 months | 25 °C / 60 % RH, PVC/AI, 2 months | 40 °C / 75 % RH, PVC/AI, 3 months | 25 °C / 60 % RH, PVC/AI, 3 months |
| **Example 8** | 0.018 | 0.018 | 0.014 | 0.016 |
| **Example 9** | 0.045 | 0.051 | 0.045 | 0.061 |
| **Example 10** | 0.010 | 0.011 | < 0.010 | < 0.010 |
| **Example 11** | 0.016 | 0.017 | 0.014 | 0.015 |

### Formulation of Metformin HCl with an anti-oxidant, free radical scavenger or reducing agent

The inventors investigated the effect of anti-oxidants, free radical scavengers or reducing agents on NDMA formation in a finished dosage form comprising metformin HCl.

### Example 12 (Comparative Example)

The batch was prepared as a comparative example with no anti-oxidant, free radical scavenger or reducing agent. The batch was prepared substantially as described in the general preparation procedure above. Metformin HCl containing 228 ppm of DMA impurity was used as the starting material.

### Example 13

Metformin HCl containing 228 ppm of DMA impurity was used as the starting material. The batch was prepared using sodium sulphite, as described below.

Metformin HCl (400.0 g) was loaded into a high shear granulator and chopped for 20 minutes. Hypromellose 15 MPa-s (14.08 g) was added and chopped with the metformin HCl for an additional 10 minutes. Sodium sulphite 0.1% (0.4 g) was dissolved in a solution of povidone K25 (21.20 g) in water (30.00 g). The solution was pumped into the high shear granulator over a 9 minute period and kneaded for an additional 4 minutes. The obtained granules were dried using fluid-bed drying, to achieve a water content of below 1.4%. The granules were then sieved, lubricated with magnesium stearate (2.32 g) and subjected to compression (12.0 mm diameter, 20R, tablet thickness, 5.10 mm, 547 mg/tablet). The tablets were then film-coated with a suspension of Hypromellose 5 MPa-s (5.20 g), macrogol 6000 (1.04 g) and titanium dioxide (4.16 g) in water (57 g) using a pan coater. The amount of coating in each individual tablet was 13 mg. The amount of DMA in the drug product was 10 ppm. The amount of sodium sulphite is 0.5 mg in each individual tablet.

**Table 6: The NDMA content of batches containing metformin HCl with the same amount of DMA impurity formulated with or without sodium sulphite, measured after 7 days storage at 60 °C and 75 % relative humidity**

| | **NDMA Content (ppm)** |
|---|---|
| **Batch** | 60 °C / 75 % RH, AI/AI, 7 days |
| **Example 12** | 0.062 |
| **Example 13** | 0.022 |

### Formulation of Metformin HCl with a pH modifying agent

The inventors investigated the effect of pH modifying agents on NDMA formation in a finished dosage form comprising metformin HCl.

### Example 14 (Comparative Example)

Metformin HCl containing 228 ppm of DMA impurity was used as the starting material. The batch was prepared as a comparative example with no pH modifying agent, substantially as described in the general preparation procedure above.

### Example 15

Metformin HCl containing 228 ppm of DMA impurity was used as the starting material. The batch was prepared, using sodium carbonate, as described below:
Metformin HCl (400.0 g) was loaded into a high shear granulator and chopped for 20 minutes. Hypromellose 15 MPa-s (14.08 g) was added and chopped with the metformin HCl for an additional 10 minutes. Sodium carbonate (10.0 g) was added and chopped for a further 5 minutes. A solution of povidone K25 (21.20 g) in water (30.00 g) was pumped into the high shear granulator over a 9 minute period and kneaded for an additional 4 minutes. The obtained granules were dried using fluid-bed drying, to achieve a water content of below 1.4%. The granules were then sieved, lubricated with magnesium stearate (2.32 g) and subjected to compression (12.0 mm diameter, 20R, tablet thickness, 5.10 mm, 547 mg/tablet). The tablets were then film-coated with a suspension of Hypromellose 5 MPa-s (5.20 g), macrogol 6000 (1.04 g) and titanium dioxide (4.16 g) in water (57 g) using a pan coater. The amount of coating in each individual tablet was 13 mg. The amount of DMA in the drug product was 10 ppm.

**Table 7: DMA content of batches of metformin HCl formulated with or without sodium carbonate**

| **Batch** | **DMA (ppm)** |
|---|---|
| **Example 14** | 215 |
| **Example 15** | 10 |

### Examples 16 to 20

The inventors further investigated the effect of the amount of pH modifying agent has on NDMA formation in a finished dosage form comprising metformin HCl. Five batches of tablets were prepared, with the amount of the sodium carbonate varying between 2.5 mg and 12.5 mg per tablet. Examples 16 to 20 were prepared substantially as described in Example 15 above, varying only in the amount of sodium carbonate used for each tablet. Metformin HCl containing 228 ppm of DMA impurity was used as the starting material.

The DMA and NDMA content of the batches (Examples 16 to 20) was measured. Measurements were taken immediately after formulation (T0) and after 7 days storage at 60 °C and 75 % relative humidity. The results are summarised in Table 8.

**Table 8: The NDMA and DMA content of batches containing metformin HCl formulated with varying amounts of sodium carbonate, measured immediately after formulation (T0) and after 7 days storage at 60 °C and 75 % relative humidity**

| | | **NDMA Content (ppm)** | | **DMA Content (ppm)** | |
|---|---|---|---|---|---|
| **Batch** | **Sodium Carbonate (mg)** | T0 | 60 °C / 75 % RH, AI/AI, 7 days | T0 | 60 °C / 75 % RH, AI/AI, 7 days |
| **Example 16** | 12.5 | < 0.010 | < 0.010 | 12 | 9 |
| **Example 17** | 10.0 | < 0.010 | < 0.010 | 12 | 9 |
| **Example 18** | 7.5 | < 0.010 | < 0.010 | 11 | 9 |
| **Example 19** | 5.0 | < 0.010 | < 0.010 | 14 | 9 |
| **Example 20** | 2.5 | < 0.010 | < 0.010 | 17 | 9 |

### Example 21

The inventors further investigated the effect of the amount of the pH modifying agent sodium bicarbonate has on NDMA formation in a finished dosage form comprising metformin HCl.

The batch was prepared substantially as described in Example 15 above, varying only in that the pH modifying agent was sodium bicarbonate. 7.5 g of sodium bicarbonate used for each tablet. Metformin HCl containing 228 ppm of DMA impurity was used as the starting material.

The DMA and NDMA content of Example 21 was then measured. The DMA and NDMA content of the batch was measured immediately after formulation (T0) and after 7 days storage at 60 °C and 75 % relative humidity. The results are summarised in Table 9.

**Table 9: The NDMA and DMA content of batches containing metformin HCl formulated with varying amounts of sodium bicarbonate, measured immediately after formulation (T0) and after 7 days storage at 60 °C and 75 % relative humidity**

| | | **NDMA Content (ppm)** | | **DMA Content (ppm)** | |
|---|---|---|---|---|---|
| **Batch** | **Sodium Bicarbonate (mg)** | T0 | 60 °C / 75 % RH, AI/AI, 7 days | T0 | 60 °C / 75 % RH, AI/AI, 7 days |
| **Example 21** | 7.5 | < 0.010 | < 0.010 | 34 | 10 |

### Combined Pharmaceutical Preparations of Sitaaliptin and Metformin

### General Procedure

Metformin hydrochloride, Sitagliptin phosphate monohydrate, cellulose microcrystalline and povidone K30 were sieved and mixed together. The resulting mixture was granulated with a solution of sodium sulfite in water in a fluid-bed granulator. The resulting granulate was dried, sieved and homogenised with additional excipients, sodium stearyl fumarate and sodium lauryl sulphate. The resulting tableting mixture was compressed on a high speed rotary tableting machine into oblong cores. The cores were coated with a cosmetic coating.

**Table 10: A combined pharmaceutical preparation of Sitagliptin/Metformin 50/1000 mg according to the present invention made according to the general procedure described above**

| **Sitagliptin/Metformin 50/1000 mg, FCT** | **Amount (mg)** | **Percentage (%)** |
|---|---|---|
| Sitagliptin phosphate monohydrate | 64.24 | 4.82 |
| Metformin hydrochloride | 1000.00 | 75.04 |
| Cellulose microcrystalline | 110.96 | 8.33 |
| Povidone K30 | 91.00 | 6.83 |
| Sodium sulfite | 1.30 | 0.10 |
| Sodium stearyl fumarate | 26.00 | 1.95 |
| Sodium lauryl sulphate | 6.50 | 0.49 |
| Total core: | **1300.00** | **97.55** |
| Coating (Opadry II) | 32.50 | 2.44 |
| Total: | **1332.50** | **100.00** |

**Table 11: A combined pharmaceutical preparation of Sitagliptin/Metformin 50/850 mg according to the present invention made according to the general procedure described above**

| **Sitagliptin/Metformin 50/850 mg, FCT** | **Amount (mg)** | **Percentage (%)** |
|---|---|---|
| Sitagliptin phosphate monohydrate | 64.24 | 5.61 |
| Metformin hydrochloride | 850.00 | 74.24 |
| Cellulose microcrystalline | 95.54 | 8.34 |
| Povidone K30 | 78.20 | 6.83 |
| Sodium sulfite | 1.12 | 0.10 |
| Sodium stearyl fumarate | 22.30 | 1.95 |
| Sodium lauryl sulphate | 5.60 | 0.49 |
| Total core: | **1117.00** | **97.55** |
| Coating (Opadry II) | 28.00 | 2.45 |
| Total: | **1145.00** | **100.00** |

### NDMA Content of Combined Pharmaceutical Preparations of Sitaaliptin and Metformin

The DMA and NDMA content of combined pharmaceutical preparations of Sitagliptin/Metformin 50/1000 mg (see **Table 10)** and Sitagliptin/Metformin 50/850 mg (see **Table 11)** [made by fluid-bed granulation (FGB) and high speed granulation (HSG)] were measured after 7 days storage at 60 °C and 75 % relative humidity. The Reference compositions were made according to the general procedure described above [by fluid-bed granulation (FGB) and high speed granulation (HSG)], but do not include sodium sulfite in the composition. The results are shown in Table 12.

**Table 12: The NDMA and DMA content of Sitagliptin/Metformin 50/1000 mg and Sitagliptin/Metformin 50/850 mg measured at T0 and after 7 days storage at 60 °C and 75 % relative humidity.**

| | | T0 (PVDC120/PVDC90) | | | 60°C/75%RH, PVDC120/PVDC90 7 days | | |
|---|---|---|---|---|---|---|---|
| **Sample** | **Composition** | **NDMA (ppm)** | **DMA (ppm)** | **pH** | **NDMA (ppm)** | **DMA (ppm)** | **pH** |
| Sitagliptin/Metformin 50/1000 mg, FCT | Reference FGB | < 0.010 | 192 | 6.3 | 0.025 | 231 | 6.5 |
| Sitagliptin/Metformin 50/1000 mg, FCT | Na₂SO₃ 0.1% FGB | < 0.010 | 194 | 6.4 | < 0.010 | 239 | 6.5 |
| Sitagliptin/Metformin 50/1000 mg, FCT | Reference HSG | < 0.010 | 209 | 6.3 | 0.032 | 237 | 6.4 |
| Sitagliptin/Metformin 50/1000 mg, FCT | Na₂SO₃ 0.1% HSG | < 0.010 | 198 | 6.4 | < 0.010 | 238 | 6.5 |
| Sitagliptin/Metformin 50/850 mg, FCT | Reference FGB | < 0.010 | 205 | 6.2 | 0.025 | 238 | 6.5 |
| Sitagliptin/Metformin 50/850 mg, FCT | Na₂SO₃ 0.1% FGB | < 0.010 | 206 | 6.4 | < 0.010 | 244 | 6.4 |
| Sitagliptin/Metformin 50/850 mg, FCT | Reference HSG | < 0.010 | 199 | 6.3 | 0.025 | 231 | 6.4 |
| Sitagliptin/Metformin 50/850 mg, FCT | Na₂SO₃ 0.1% HSG | < 0.010 | 197 | 6.3 | < 0.010 | 228 | 6.4 |

### Combined Pharmaceutical Preparations of Linaaliptin and Metformin

### General Procedure

Metformin hydrochloride, Linagliptin, maize starch and copovidone were sieved and mixed together. The resulting mixture was granulated with a solution of glycerine and sodium hydroxide in water in a high shear mixer. The resulting granulate was dried, sieved and homogenised with additional excipients, silica, colloidal anhydrous and magnesium stearate. The resulting tableting mixture was compressed on a high speed rotary tableting machine into oblong cores. The cores were coated with a cosmetic coating.

**Table 13: A combined pharmaceutical preparation of Linagliptin/Metformin 2.5/850 mg according to the present invention made according to the general procedure described above**

| **Linagliptin/Metformin 2.5/850 mg, FCT** | **Amount (mg)** | **Percentage (%)** |
|---|---|---|
| Linagliptin | 2.5 | 0.25 |
| Metformin hydrochloride | 850.00 | 82.49 |
| Maize starch | 33.10 | 3.21 |
| Copovidone | 80.50 | 7.81 |
| Glycine | 21.25 | 2.06 |
| Sodium hydroxide | 10.20 | 0.99 |
| Silica, colloidal anhydrous | 4.20 | 0.41 |
| Magnesium stearate | 8.50 | 0.82 |
| Total of cores: | **1010.25** | **98.04** |
| Coating | 20.21 | 1.96 |
| Total: | **1030.46** | **100.00** |

**Table 14: A combined pharmaceutical preparation of Linagliptin/Metformin 2.5/1000 mg according to the present invention made according to the general procedure described above**

| **Linagliptin/Metformin 2.5/1000 mg, FCT** | **Amount (mg)** | **Percentage (%)** |
|---|---|---|
| Linagliptin | 2.50 | 0.21 |
| Metformin hydrochloride | 1000.00 | 82.24 |
| Maize starch | 42.50 | 3.50 |
| Copovidone | 95.00 | 7.81 |
| Glycine | 25.00 | 2.06 |
| Sodium hydroxide | 12.00 | 0.99 |
| Silica, colloidal anhydrous | 5.00 | 0.41 |
| Magnesium stearate | 10.00 | 0.82 |
| Total of cores: | **1192.00** | **98.03** |
| Coating | 23.90 | 1.97 |
| Total: | **1215.90** | **100.00** |

The invention includes the subject matter described in the following numbered paragraphs:
1. A pharmaceutical composition comprising an active pharmaceutical ingredient or salt thereof, wherein the composition comprises less than 100 ppm of peroxide impurities and more than 50 ppm of an amine impurity.
2. The pharmaceutical composition according to paragraph 1 comprising a low peroxide grade excipient.
3. The pharmaceutical composition according to paragraph 2 wherein the low peroxide grade excipient is povidone K25 or povidone LP30.
4. The pharmaceutical composition according to any one of paragraphs 1 to 3 comprising at least one of an anti-oxidant, free radical scavenger or reducing agent.
5. The pharmaceutical composition according to paragraph 4 wherein the anti-oxidant, free radical scavenger or reducing agent is sodium sulphite.
6. The pharmaceutical composition according to any one of paragraphs 4 to 5 wherein the anti-oxidant, free radical scavenger or reducing agent active is integrated in a low peroxide grade excipient.
7. The pharmaceutical composition according to any one of paragraphs 1 to 6 wherein the active pharmaceutical ingredient or salt thereof is metformin hydrochloride.
8. The pharmaceutical composition according to any one of paragraphs 1 to 7 wherein the amine impurity is dimethylamine.
9. A pharmaceutical composition comprising an active pharmaceutical ingredient or salt thereof and a pH modifying agent, wherein the composition comprises more than 50 ppm of peroxide impurities and less than 50 ppm of an amine impurity.
10. The pharmaceutical composition according to paragraph 9 wherein the active pharmaceutical ingredient or salt thereof is metformin hydrochloride.
11. The pharmaceutical composition according to any one of paragraphs 9 to 10 wherein the pH modifying agent is a base, preferably sodium carbonate, sodium hydrogen carbonate or sodium bicarbonate.
12. The pharmaceutical composition according to any one of paragraphs 9 to 11 wherein the amine impurity is dimethylamine.
13. A method for reducing the amount of an amine impurity in an active pharmaceutical ingredient or salt thereof, the method comprising:
   a) providing a solution of the active pharmaceutical ingredient or salt thereof in a solvent;
   b) crystallising the active pharmaceutical ingredient from the solvent; and
   c) removing the solvent to obtain the pure active pharmaceutical ingredient or salt thereof.
14. The method according to paragraph 13 wherein the amine impurity is reduced to less than 50 ppm.
15. The method according to paragraph 13 or 14 wherein the active pharmaceutical ingredient or salt thereof is metformin hydrochloride.
16. The method according to any one of paragraphs 13 to 15 wherein the amine impurity is dimethylamine.
17. The method according to of any one of paragraphs 13 to 16 wherein the solvent is water, an alcoholic solvent, or a mixture thereof.
18. A method for reducing the amount of an amine impurity in an active pharmaceutical ingredient or salt thereof, the method comprising: heating the active pharmaceutical ingredient or salt thereof to a temperature between 50 and 120 °C.
19. The method according to paragraph 18 wherein the amine impurity is reduced to less than 50 ppm.
20. The method according to paragraph 18 or 19 wherein the active pharmaceutical ingredient or salt thereof is metformin hydrochloride.
21. The method according to any one of paragraphs 18 to 20 wherein the amine impurity is dimethylamine.

The invention may also be as defined in the following numbered clauses.
1. A pharmaceutical composition comprising metformin or a salt thereof and an anti-oxidant, free radical scavenger or reducing agent, wherein the composition comprises less than 100 ppm of peroxide impurities and more than 50 ppm of dimethylamine.
2. The pharmaceutical composition according to clause 1 comprising a low peroxide grade excipient.
3. The pharmaceutical composition according to clause 2 wherein the low peroxide grade excipient is povidone K25 or povidone LP30.
4. The pharmaceutical composition according to any one of clauses 1 to 3 wherein the anti-oxidant, free radical scavenger or reducing agent is sodium sulphite.
5. The pharmaceutical composition according to any one of clauses 1 to 4 wherein the anti-oxidant, free radical scavenger or reducing agent active is integrated in a low peroxide grade excipient.
6. The pharmaceutical composition according to any one of clauses 1 to 5 for use in the treatment of type !! diabetes.
7. A combined pharmaceutical preparation comprising metformin or a salt thereof, an anti-oxidant, free radical scavenger or reducing agent and a sodium-glucose co-transporter-2 (SGLT2) inhibitor and/or a dipeptidyl peptidase-4 (DPP-4) inhibitor, wherein the composition comprises less than 100 ppm of peroxide impurities and more than 50 ppm of dimethylamine.
8. The combined pharmaceutical preparation according to clause 7 wherein the sodium-glucose co-transporter-2 (SGLT2) inhibitor is a flozin, selected from empagliflozin, dapagliflozin or canagliflozin.
9. The combined pharmaceutical preparation according to clause 7 or 8 wherein the dipeptidyl peptidase-4 (DPP-4) inhibitor is a gliptin, selected from vildagliptin, sitagliptin, saxagliptin, alogliptin or linagliptin.
10. The combined pharmaceutical composition according to any one of clauses 7 to 9 for use in the treatment of type !! diabetes.

## Claims

1. A pharmaceutical composition comprising an active pharmaceutical ingredient or salt thereof and a pH modifying agent,
wherein the composition comprises more than 50 ppm of peroxide impurities and less than 50 ppm of an amine impurity,
wherein the pH modifying agent is present in an amount from 0.1 to 5 wt% of the pharmaceutical composition, and
wherein the active pharmaceutical ingredient i) consists of metformin or a salt thereof; ii) comprises metformin or a salt thereof and a sodium-glucose co-transporter-2 (SGLT2) inhibitor or a salt thereof; or iii) comprises metformin or a salt thereof and a dipeptidyl peptidase-4 (DPP-4) inhibitor.

2. The pharmaceutical composition according to claim 1, wherein the active pharmaceutical ingredient or salt thereof comprises metformin hydrochloride.

3. The pharmaceutical composition according to claim 1 or 2, wherein the pH modifying agent is selected from the group consisting of an acid, a base or a buffer.

4. The pharmaceutical composition according to claim 3, wherein the pH modifying agent is a base selected from the group consisting of a carbonate or hydrogen carbonate salt of an alkali or alkaline earth metal.

5. The pharmaceutical composition according to claim 4, wherein the pH modifying agent is selected from the group consisting of sodium carbonate, sodium bicarbonate, sodium hydrogen carbonate or combination thereof.

6. The pharmaceutical composition according to any preceding claim, wherein the pH modifying agent is present in an amount from 0.2 to 4 wt%, 0.3 to 3 wt%, 0.4 to 2 wt% or 0.5 to 1 wt% of the pharmaceutical composition.

7. The pharmaceutical composition according to any preceding claim, wherein the sodium-glucose co-transporter-2 (SGLT2) inhibitor is a flozin, preferably selected from empagliflozin, dapagliflozin or canagliflozin.

8. The pharmaceutical composition according to claim 7, wherein the sodium-glucose co-transporter-2 (SGLT2) is dapagliflozin or a salt thereof.

9. The pharmaceutical composition according to any one of claims 1 to 6, wherein the dipeptidyl peptidase-4 (DPP-4) inhibitor is a gliptin, preferably selected from vildagliptin, sitagliptin, saxagliptin, alogliptin or linagliptin.

10. The pharmaceutical composition according to any preceding claim for use in the treatment of type !! diabetes.

11. A method for reducing the amount of an amine impurity in a pharmaceutical composition according to any of claims 1 to 9, the method comprising:
c) mixing the active pharmaceutical ingredient or salt thereof with a pH modifying agent; and
d) formulating the mixture of a) into a finished dosage form.
